(19) Europäisches Patentamt
European Patent Office
Office européen des brevets

(11) **EP 3 984 492 B1**

(12) **EUROPÄISCHE PATENTSCHRIFT**

(45) Veröffentlichungstag und Bekanntmachung des Hinweises auf die Patenterteilung:
**04.10.2023 Patentblatt 2023/40**

(21) Anmeldenummer: **20215936.4**

(22) Anmeldetag: **21.12.2020**

(51) Internationale Patentklassifikation (IPC):
**A61C 5/73** (2017.01)     **A61C 5/77** (2017.01)
**A61C 13/00** (2006.01)     **A61C 13/08** (2006.01)
**B33Y 80/00** (2015.01)

(52) Gemeinsame Patentklassifikation (CPC):
**A61C 5/77; A61C 5/73; A61C 13/0004;**
**A61C 13/08; A61C 13/082; A61C 13/09;**
**B33Y 50/00; B33Y 80/00**

(54) **VERGLEICH VON FARBBILDERN DREIDIMENSIONALER DENTALER STRUKTUREN**

COMPARISON OF COLOUR IMAGES OF THREE-DIMENSIONAL DENTAL STRUCTURES

COMPARAISON DES IMAGES COULEURS DES STRUCTURES DENTAIRES TRIDIMENSIONNELLES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2020 EP 20201553**

(43) Veröffentlichungstag der Anmeldung:
**20.04.2022 Patentblatt 2022/16**

(73) Patentinhaber: **Ivoclar Vivadent AG**
**9494 Schaan (LI)**

(72) Erfinder:
• **JUSSEL, Rudolf**
**6805 Feldkirch-Gisingen (AT)**

• **NIEDRIG, Christian**
**9464 Rüthi (CH)**
• **JOHN, Hendrik**
**9470 Buchs (CH)**

(74) Vertreter: **Baldus, Oliver**
**Splanemann**
**Rumfordstrasse 7**
**80469 München (DE)**

(56) Entgegenhaltungen:
**EP-A1- 3 530 232      WO-A1-2019/023461**
**DE-A1- 19 922 870**

**Beschreibung**

Die vorliegende Erfindung betrifft ein Verfahren zum Vergleichen dreidimensionaler dentaler Strukturen, eine Computervorrichtung zum Vergleichen dreidimensionaler dentaler Strukturen und ein Computerprogramm.

Für die naturgetreue Herstellung einer dentalen Restauration ist es erforderlich, Abweichungen in einem Erscheinungsbild der künstlichen dentalen Restauration und einem natürlichen Zahn des Patienten exakt zu bestimmen. Nur wenn Abweichungen präzise ermittelt werden, ist es möglich die dentale Restauration so anzupassen und herzustellen, dass diese dem Erscheinungsbild des natürlichen Zahns entspricht.

Die Druckschrift DE 199 22 8870 A1 betrifft ein Verfahren zur automatischen individuellen Farb-, Transluzenz-, Helligkeits- und Fluoreszenzgebung von zahntechnischen Restaurationen.

Die Druckschrift EP 3 530 232 A2 betrifft ein computerimplementiertes Verfahren zum Ausrichten eines dreidimensionalen Modells des Gebisses eines Patienten an einem von einer Kamera aufgenommenen Bild des Gesichts des Patienten.

Die Druckschrift WO2019/023461 A1 betrifft ein Verfahren zum Herstellen einer Zahnrestauration für einen Patienten, sodass die Zahnrestauration optische Eigenschaften aufweist, die den optischen Eigenschaften der Zähne des Patienten entsprechen. Hierzu wird ein Zwischenmodel (Intermediate Model) mit Startwerten für die optischen Eigenschaften (Starting Optical Property Values) gebildet.

Es ist die technische Aufgabe der vorliegenden Erfindung, einen zuverlässigen Wert für eine Abweichung zwischen einer virtuellen Zahnstruktur und einem Abbild einer natürlichen Zahnstruktur zu bestimmen.

Diese technische Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch ein Verfahren zum Vergleichen dreidimensionaler dentaler Strukturen gelöst, mit den Schritten eines Erfassens eines Soll-Datensatzes, der die Form und die optischen Eigenschaften eines natürlichen Zahns wiedergibt; eines Renderns eines digitalen Zahnmodells zum Erzeugen eines Ist-Datensatzes, der die Form und die optischen Eigenschaften des digitalen Zahnmodells wiedergibt; eines Bestimmens eines ersten Vergleichswertes aus dem Ist-Datensatz; eines Bestimmens eines zweiten Vergleichswertes aus dem Soll-Datensatz; und eines Bestimmens einer Abweichung auf Basis des ersten und des zweiten Vergleichswertes. Der Soll-Datensatz wird so skaliert, dass eine Überlagerung mit dem Ist-Datensatz im gleichen Koordinatensystem erzielt wird oder der Ist-Datensatz wird so skaliert, dass eine Überlagerung mit dem Soll-Datensatz im gleichen Koordinatensystem erzielt wird. Die ersten und zweiten Vergleichswerte werden aus einem Bereich des Koordinatensystems erhalten. Bei dem Bereich kann es sich um eine rechteckige oder kreisförmige Fläche des Koordinatensystems handeln. Dadurch können Abweichungen in vorgegebenen Flächenbereichen ermittelt und quantifiziert werden. Dadurch wird beispielsweise der technische Vorteil erreicht, dass bestimmte Bildpunkte zu Flächen zusammengefasst werden können und so der Farbabgleich vereinfacht und beschleunigt werden kann, dass sich genaue Werte für die Abweichung über einen ganzen Bereich ermitteln lassen. Die Skalierung kann auf Basis der Form aus dem Ist-Datensatz und/oder dem Soll-Datensatz durchgeführt werden. Dadurch wird beispielsweise der technische Vorteil erreicht, dass sich die Vergleichswerte einfach und zuverlässig ermitteln lassen.

Durch ein Vergleichen einer Differenz an einem Bildpunkt oder einer reduzierten Anzahl an Bildpunkten kann das Rendering auf diese Bildpunkte beschränkt werden, so dass erhebliche Geschwindigkeitsvorteile entstehen. Zum Zweck der Vereinfachung der Vergleichsmessung können zweidimensionale Bilder des natürlichen Zahns und des digitalen Zahnmodells abgeleitet werden, die denselben Betrachtungswinkel aufweisen und die Vergleichswerte aus der Überlagerung der zweidimensionalen Farbbilder bestimmt werden.

In einer weiteren technisch vorteilhaften Ausführungsform des Verfahrens wird die Abweichung auf Basis mehrerer erster und zweiter Vergleichswerte an unterschiedlichen Positionen des Koordinatensystems bestimmt. Hierzu können mehrere erste und zweite Vergleichswerte an den jeweiligen Positionen des Koordinatensystems des Soll-Datensatzes und des Ist-Datensatzes bestimmt werden. Dadurch wird beispielsweise der technische Vorteil erreicht, dass sich die Genauigkeit der bestimmten Abweichung verbessert.

In einer weiteren technisch vorteilhaften Ausführungsform des Verfahrens werden die ersten und zweiten Vergleichswerte entlang eines Rasters oder entlang von Linien innerhalb des Koordinatensystems erhalten. Dadurch wird beispielsweise der technische Vorteil erreicht, dass sich Vergleichswerte mit wenigen Schritten aus dem gesamten Bereich ermitteln lassen.

In einer weiteren technisch vorteilhaften Ausführungsform des Verfahrens wird die Abweichung auf Basis eines euklidischen Abstands des ersten und des zweiten Vergleichswerts bestimmt. Dadurch wird beispielsweise der technische Vorteil erreicht, dass ein standardisiertes und präzises Maß für die Abweichung erhalten wird.

In einer weiteren technisch vorteilhaften Ausführungsform des Verfahrens wird ein Bildrauschen des Soll-Datensatzes und/oder des Ist-Datensatzes durch ein Filter reduziert. Dadurch wird beispielsweise der technische Vorteil erreicht, dass ein Bildrauschen reduziert werden kann und größere Flächen besser verglichen werden können.

In einer weiteren technisch vorteilhaften Ausführungsform des Verfahrens umfasst der Soll-Datensatz und/oder der Ist-Datensatz ein zweidimensionales Bild. Dadurch wird beispielsweise der technische Vorteil erreicht, dass die Abweichung mit geringem Rechenaufwand ermittelt werden kann.

In einer weiteren technisch vorteilhaften Ausführungsform des Verfahrens umfasst der Soll-Datensatz und/oder der Ist-Datensatz mehrere zweidimensionale Bilder aus mehreren Blickwinkeln. Dadurch wird beispielsweise der technische Vorteil erreicht, dass die Genauigkeit des Verfahrens erhöht wird.

In einer weiteren technisch vorteilhaften Ausführungsform des Verfahrens wird die Abweichung auf Basis mehrerer erster und zweiter Vergleichswerte in unterschiedlichen zweidimensionalen Bildern bestimmt. Dadurch wird beispielsweise der technische Vorteil erreicht, dass die Genauigkeit des Verfahrens noch weiter verbessert wird.

In einer weiteren technisch vorteilhaften Ausführungsform des Verfahrens umfasst der Soll-Datensatz und/oder der Ist-Datensatz eine dreidimensionale Form. Dadurch wird beispielsweise der technische Vorteil erreicht, dass sich die Abweichung über die gesamte Form vergleichen lässt.

In einer weiteren technisch vorteilhaften Ausführungsform des Verfahrens wird die Abweichung auf Basis mehrerer erster und zweiter Vergleichswerte an den jeweils gleichen Positionen auf der Oberfläche der dreidimensionalen Form bestimmt. Dadurch wird beispielsweise der technische Vorteil erreicht, dass die Abweichung über den gesamten Zahn ermittelt werden kann.

In einer weiteren technisch vorteilhaften Ausführungsform des Verfahrens ist das Koordinatensystem ein zweidimensionales oder ein dreidimensionales Koordinatensystem. Dadurch wird beispielsweise der technische Vorteil erreicht, dass besonders geeignete Koordinatensysteme verwendet werden und die Bildpunkte in dem Soll- und Ist-Datensatz präzise referenziert werden können.

In einer weiteren technisch vorteilhaften Ausführungsform des Verfahrens wird der Soll-Datensatz mittels einer elektronischen Kamera oder einem 3D-Scanner erfasst. Die elektronische Kamera oder der 3D-Scanner bilden einen Sensor zum Erfassen des Soll-Datensatzes. Dadurch wird beispielsweise der technische Vorteil erreicht, dass der Soll-Datensatz auf einfache und schnelle Weise erhalten werden kann.

Gemäß einem zweiten Aspekt wird die technische Aufgabe durch eine Computervorrichtung zum Vergleichen dreidimensionaler dentaler Strukturen gelöst, mit einem Sensor zum Erfassen eines Soll-Datensatzes auf Basis eines natürlichen Zahns, die geeignet ist, das Verfahren nach dem ersten Aspekt auszuführen. Dadurch werden die gleichen technischen Vorteile wie durch das Verfahren nach dem ersten Aspekt erreicht.

Gemäß einem dritten Aspekt wird die technische Aufgabe durch ein Computerprogramm gelöst, umfassend Befehle, die bewirken, dass die Computervorrichtung nach dem zweiten Aspekt das Verfahren nach dem ersten Aspekt ausführt. Dadurch werden die gleichen technischen Vorteile wie durch das Verfahren nach dem ersten Aspekt erreicht.

Ausführungsbeispiele der Erfindung sind in den Zeichnungen dargestellt und werden im Folgenden näher beschrieben.

Es zeigen:

Fig. 1 eine schematische Ansicht einer Erfassung eines Soll-Datensatzes und eines Ist-Datensatzes;

Fig. 2 eine schematische Ansicht einer Erfassung des Soll-Datensatzes und eines Ist-Datensatzes aus mehreren Blickwinkeln;

Fig. 3 eine schematische Ansicht einer Skalierung des Soll-Datensatzes auf den Ist-Datensatz;

Fig. 4 eine schematische Ansicht mehrerer Verfahren zum Berechnen einer Abweichung aus aufeinander-skalierten Datensätzen;

Fig. 5 eine Ansicht unterschiedlicher Differenzwerte, die über einen flächigen Bereich ermittelt worden sind;

Fig. 6 Farbdifferenzen zwischen einem Soll-Datensatz und einem Ist-Datensatz;

Fig. 7 Farbdifferenzen zwischen einem weiteren Soll-Datensatz und einem weiteren Ist-Datensatz; und

Fig. 8 ein Blockdiagramm eines Verfahrens zum Vergleichen dreidimensionaler dentaler Strukturen.

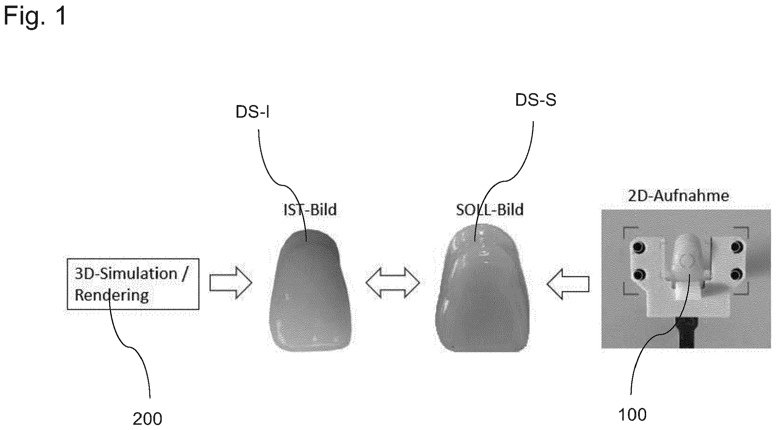

Fig. 1 zeigt eine schematische Ansicht einer Erfassung eines Soll-Datensatzes DS-S und eines Ist-Datensatzes DS-I. Der Soll-Datensatz DS-S gibt die Form und die optischen Eigenschaften eines natürlichen Zahns 100 zwei- oder dreidimensional wieder. Der Soll-Datensatz DS-S wird beispielsweise durch eine elektronische kalibrierte Kamera 103 erfasst, die die sichtbaren Bereiche des Zahns 100 eines Patienten erfasst. Diese normierten Farbwerte der Kamera im Soll-Datensatz DS-S bilden später die gemeinsame Basis für einen Soll/Ist-Vergleich. Bei Einsatz einer Kamera wird vorzugsweise das Bild im RAW-Format verwendet, durch das die direkten Messwerte des Sensors genutzt werden. Dieses Format kann dann in den L*a*b*-Farbraum transformiert werden. Aus dem Soll-Datensatz DS-S kann dann ein Soll-Bild ermittelt werden, an das das äußere Erscheinungsbild der dentalen Restauration angepasst wird.

Die Erfassung des realen Zahns kann mittels eines Intraoralscanners erfolgen. In diesem Fall ist die Farbinformation zusammen mit einer dreidimensionalen Form in dem Soll-Datensatz DS-S verknüpft. Aus den dreidimensionalen Soll-Datensatz DS-S können dann Soll-Bilder des Zahns abgeleitet werden. Der Soll-Datensatz DS-S kann aber auch ein oder mehrere Bilder umfassen.

Das digitale Zahnmodell 200 gibt demgegenüber eine äußere Form und eine innere Struktur einer dentalen Restauration wieder, die auf einem Planungsentwurf beruht. Die innere Struktur der dentalen Restauration kann mehrere Schichten aus unterschiedlichen Restaurationsmaterialien für den Aufbau umfassen. Beispielsweise lassen sich räumliche Bereiche für eine Schneide der dentalen Restauration und einen Dentinkern innerhalb der dentalen Restauration festlegen. Zusätzlich kann auch eine Präparation des verbliebenen, natürlichen Zahns und eine Befestigungsschicht berücksichtigt werden, wie beispielsweise ein Zement oder ein Adhäsiv. Die physikalisch optischen Parameter der zugewiesenen Restaurationsmaterialien sind bekannt.

Der gesamte Aufbau des digitalen Zahnmodells 200 wird einer Rendering-/Simulations-Software einschließlich der Zuordnung der physikalisch optischen Materialeigenschaften zu den verschiedenen räumlichen Bereichen, Schichten oder Subvolumen übergeben. Daraus erzeugt die Rendering-/Simulations-Software nun einen Ist-Datensatz DS-I, aus dem Ist-Bilder mit normierten Farb- und Transluzenz-Werten abgeleitet werden können. Der Ist-Datensatz DS-I gibt somit die Form und die optischen Eigenschaften eines digitalen Zahnmodells 200 für eine geplante dentale Restauration wieder. Der Ist-Datensatz DS-I kann aber auch ein oder mehrere Bilder umfassen.

Das Rendern wird mittels einer physikalisch korrekten Simulation der Wechselwirkung von Licht und der geplanten dentalen Restauration 100 und den verwendeten Restaurationsmaterialien durchgeführt. Dabei werden die bekannten optischen Parameter der einzelnen Restaurationsmaterialien verwendet, um eine computergestützte Ansicht der dentalen Restauration 100 zu erzeugen.

Hierzu kann zusätzlich bestehendes natürliches Zahnmaterial berücksichtigt werden, wie beispielsweise ein Restzahn, auf dem die dentale Restauration 100 aufgesetzt werden soll. Beim Rendern wird zu dem vorgegebenen inneren Aufbau und den ausgewählten Restaurationsmaterialien der optische Eindruck der späteren dentalen Restauration 100 errechnet. Für das Rendern kann computergestützt eine Berechnung von Reflexions-, Transmissions- und Absorptionswerten im sichtbaren Bereich bei mind. drei Wellenlängen durchgeführt werden.

Der Soll-Datensatz DS-S und der Ist-Datensatz DS-I können anschließend miteinander verglichen werden, um Abweichungen zwischen der geplanten dentalen Restauration und dem entsprechenden Natürlichen Zahn festzustellen.

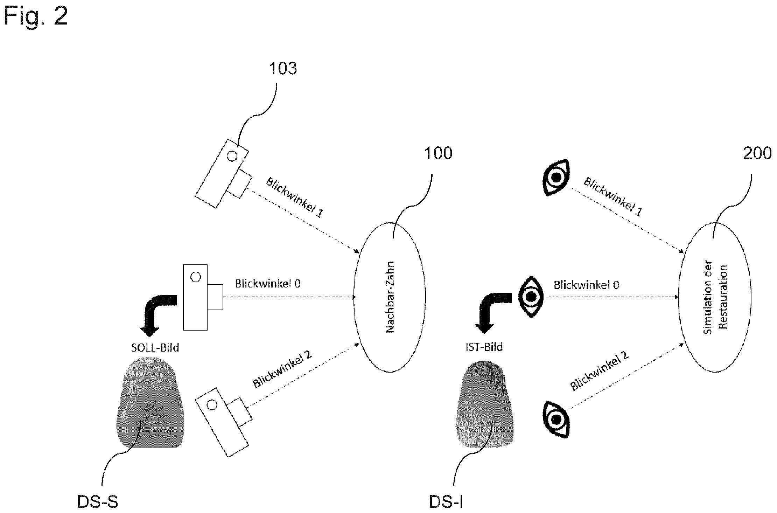

Fig. 2 zeigt eine schematische Ansicht einer Erfassung eines Soll-Datensatzes DS-S und eines Ist-Datensatzes DS-I aus mehreren Blickwinkeln.

Der Soll-Datensatz DS-S kann aus Farbaufnahmen einer elektronischen Kamera 103 von einem Nachbarzahns 100 aus verschiedenen Blickwinkeln abgeleitet sein. Vorteilhaft ist es, eine Kamera 103 mit bekannter spektraler Sensitivität einzusetzen, um eine unbekannte gerätespezifische Bilddatenverarbeitung als Fehlerquelle ausschließen zu können. Für eine Erfassen des Soll-Datensatzes DS-S ist es zudem vorteilhaft, wenn eine Beleuchtungssituation der Farbaufnahmen genau bekannt ist. Hierzu kann eine einzige Lichtquelle mit einem eindeutigen Spektrum und einer eindeutigen Lichtrichtung verwendet werden. Diese kann mit einer Spiegelkugel zur Erfassung der Reflexionen bestimmt werden. Zudem kann ein Ringblitz oder Zangenblitz in einem dunklen Raum verwendet werden.

Die Lichtverhältnisse zum Zeitpunkt der Farbaufnahme sind beispielsweise durch parallele Aufnahme einer Referenzoberfläche (Graukarte) oder Verwendung einer definierten Lichtquelle (Spektrum, Intensität und Anordnung) bekannt, um annähernd normierte Farbwerte zu erhalten. Zusammen mit der Referenzfläche können auch Informationen zu einer Transluzenz des Zahns gewonnen werden.

Eine vorherige Kalibration der Kamera 103 und die Verwendung einer Referenz kann auch zur Berechnung des Abstandes während der Farberfassung vorteilhaft sein.

Eine Farbaufnahme kann in der frontalen Betrachtung unter einem Betrachtungswinkel von 0° in Richtung Gesicht oder Mund des Patienten geschehen. Zusätzlich kann eine Farbaufnahme aus Betrachtungswinkeln zischen -90° und +90° zur Orthogonalen einer labialen Zahnoberfläche oder zwischen -45° und +45° zur Orthogonalen einer labialen Zahnoberfläche erfolgen.

Idealerweise werden die Ist-Bilder des Ist-Datensatzes DS-I unter den gleichen Blickwinkeln aus dem digitalen Zahnmodell 200 abgeleitet, aus denen auch die Soll-Bilder aufgenommen worden sind. Um einen standardisierten Vergleich der Farb- und Transluzenz-Werte von Ist- und Soll-Bild durchführen zu können, können die Referenzfläche oder der Referenzkörper ebenfalls gerendert werden, so dass eine Plausibilitätsprüfung durchgeführt werden kann.

In diesem Fall erfolgt der Vergleich anhand der zweidimensionalen Bilder, die unter den verschiedenen Blickwinkeln erfasst und abgeleitet worden sind. Je mehr Farbaufnahmen als Soll-Bilder aus unterschiedlichen Blickwinkeln erhalten und mit den jeweiligen Ist-Bildern verglichen werden, desto genauer ist hinterher die Bestimmung der Abweichung im Rahmen des Soll-Ist-Vergleichs.

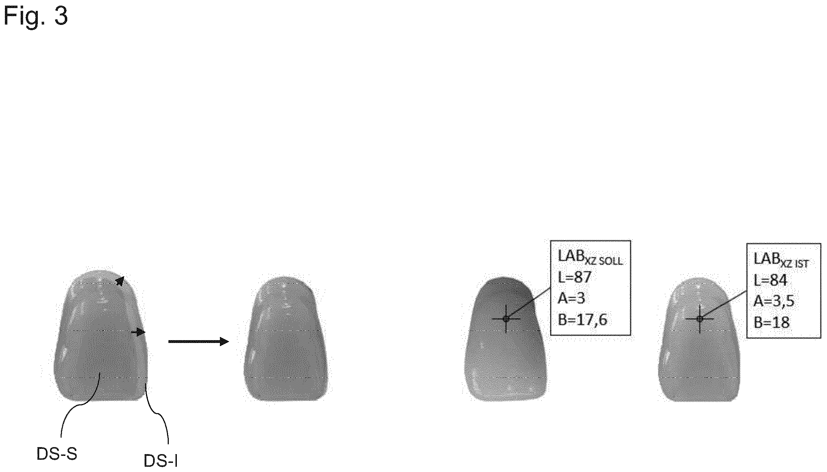

Fig. 3 zeigt eine schematische Ansicht einer Skalierung des Soll-Datensatzes DS-S auf den Ist-Datensatz DS-I. In diesem Fall wird ein Bild des Soll-Datensatzes DS-S so in Quer- und Längsrichtung gedehnt oder gestaucht (skaliert), dass sich die jeweiligen Formen des Zahns entsprechen. Der Soll-Datensatz DS-S und der Ist-Datensatz DS-I liegen anschließend in einem gemeinsamen Koordinatensystem.

Danach wird eine Position aus dem Koordinatensystem ausgewählt und die Farbwerte des Soll-Datensatzes DS-S und des Ist-Datensatzes DS-I werden an dieser Position ermittelt. In diesem Fall wird jeweils ein Farbwert $L_I^*a_I^*b_I^*$ für den Ist-Datensatz und ein Farbwert $L_S^*a_S^*b_S^*$ für den Soll-Datensatz im L*a*b-Farbraum erhalten.

Die euklidische Abweichung $\Delta E_{S,I}$ zwischen dem Soll-Datensatz DS-S und dem Ist-Datensatz DS-I kann berechnet werden, indem der Unterschiede in den Farbwerten $L_I^*a_I^*b_I^*$ und LS*aS*bS* ermittelt wird. Der Vergleich kann auf Pixel-Ebene als kleinste Auflösung erfolgen.

$$\Delta E_{S,I} = \sqrt{(L_S^* - L_I^*)^2 + (a_S^* - a_I^*)^2 + (b_S^* - b_I^*)^2}$$

Die so ermittelte Abweichung kann dann dazu verwendet werden, eine optimierte Materialzuordnung zu einer mehrschichtigen dentalen Restauration zu ermitteln um eine hoch ästhetische, patientenspezifische Restauration zu planen und in Folge dann auch zu fertigen.

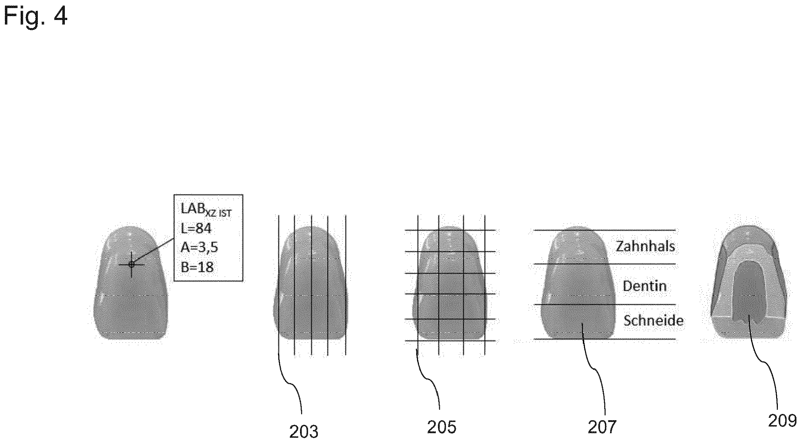

Fig. 4 zeigt eine schematische Ansicht mehrerer Verfahren zum Berechnen einer Abweichung aus aufeinanderskalierten Datensätzen DS-I und DS-S.

In einer ersten Ausführungsform wird eine einzelne Position innerhalb des Koordinatensystems bestimmt und es wird die Abweichung aus den zwei Farbwerten der Datensätze DS-I und DS-S aus einem Punkt berechnet. Diese Berechnung kann für weitere Punkte wiederholt werden.

In einer zweiten Ausführungsform wird die Abweichung entlang einer oder mehrerer Linien 203 bestimmt. Zu diesem Zweck werden die Abweichungen auf Pixelebene entlang der Linie 203 bestimmt und zu einem Gesamtwert aufsummiert. Dadurch kann die Genauigkeit des Verfahrens erhöht werden.

In einer dritten Ausführungsform wird die Abweichung entlang eines Rasters 205 mit mehreren waagerechten und senkrechten Linien bestimmt. Zu diesem Zweck werden die Abweichungen auf Pixelebene entlang den waagerechten und senkrechten Linien 203 bestimmt und ebenfalls zu einem Gesamtwert aufsummiert. Dadurch kann die Abweichung in unterschiedlichen Richtungen bestimmt werden und die Genauigkeit des Verfahrens nochmals erhöht werden.

In einer vierten Ausführungsform wird die Abweichung in bestimmten Bereichen 207 erhalten. Zu diesem Zweck werden die einzelnen Abweichungen auf Pixelebene aus einem bestimmten Bereich zu einem Wert aufsummiert. Auf diese Weise lassen sich beispielsweise Abweichungen für den Zahnhals, den Dentinbereich und die Schneide ermitteln. Die einzelnen Abweichungen lassen sich einzeln gewichten, so dass besonders relevanten Bereichen eine höhere Bedeutung für den Gesamtwert zukommt.

In einer fünften Ausführungsform wird die Abweichung aus den Bereichen 209 ermittelt, die einem bestimmten Farbschema zugeordnet werden können. Zu diesem Zweck werden die einzelnen Abweichungen auf Pixelebene aus einem bestimmten Bereich zu einem Wert aufsummiert. Auf diese Weise lassen sich beispielsweise Abweichungen für einzelne Farbbereiche ermitteln. Auch in diesem Fall lassen sich die einzelnen Abweichungen einzeln gewichten, so dass besonders relevanten Bereichen eine höhere Bedeutung für den Gesamtwert zukommt.

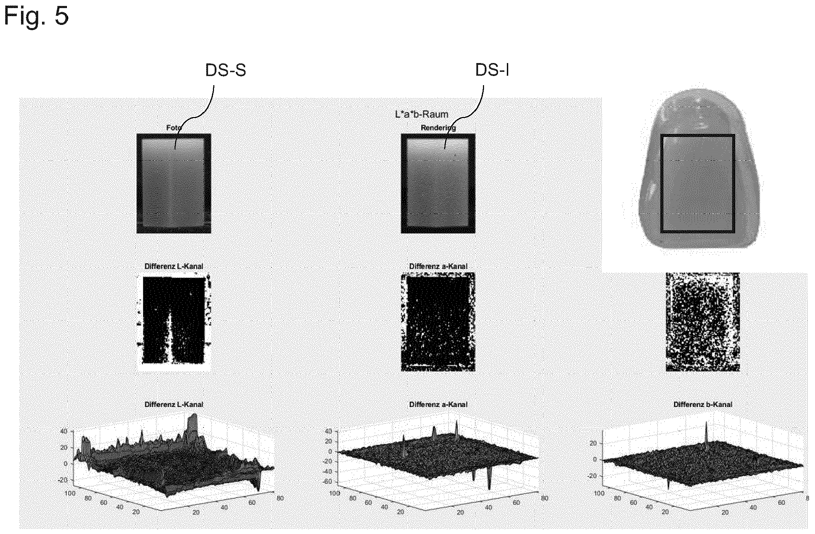

Fig. 5 zeigt eine Ansicht unterschiedlicher Differenzwerte zwischen dem Soll-Datensatz DS-S und dem Ist-Datensatz DS-I, die auf Pixelebene über einen flächigen Bereich ermittelt worden sind. In jedem der einzelnen Farbwerte im L*a*b*-Raum treten Differenzen auf, aus denen sich eine Gesamtabweichung berechnen lässt.

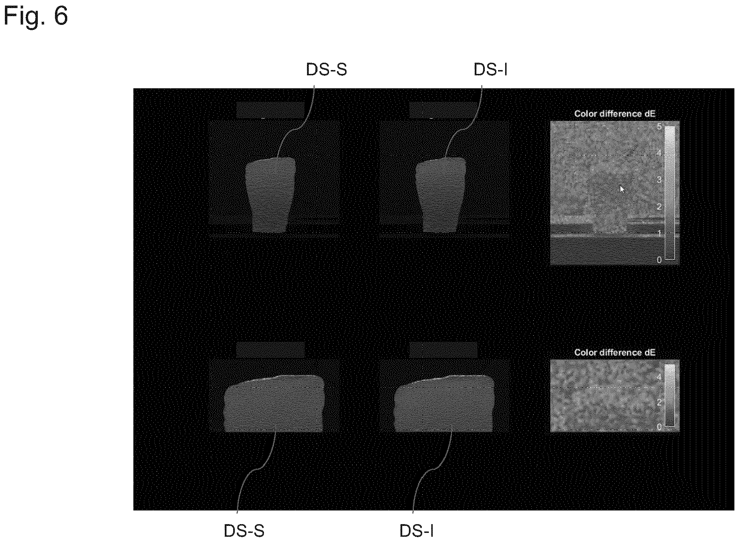

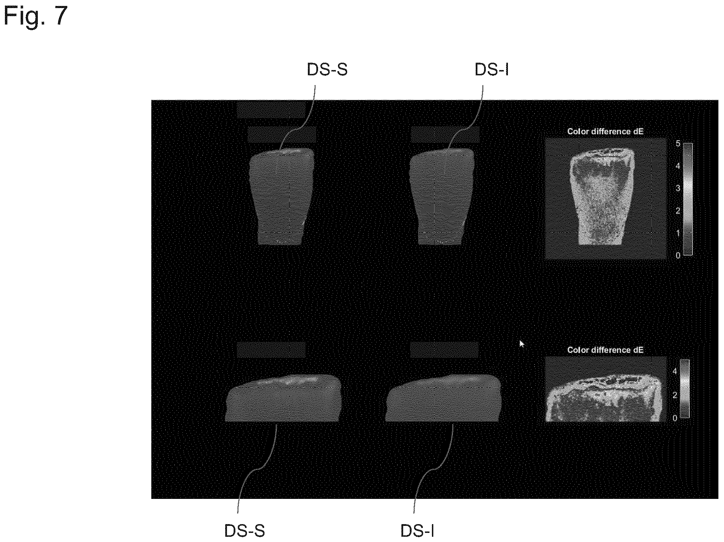

Fig. 6 und Fig. 7 zeigen Farbdifferenzen zwischen unterschiedlichen Soll-Datensätzen DS-S und Ist-Datensätzen DS-I. Aus dem Soll-Datensatz kann die gewünschte Farbverteilung und Grenzwerte ermittelt werden, wie beispielsweise die hellste und die dunkelste Farbe und die dazugehörigen LAB-Werte. Dies hat den Vorteil, dass daraus die initiale Materialzuordnung zu den unterschiedlichen Volumenbereichen des digitalen Zahnmodells bzw. der digitalen Restauration zugeordnet werden können und das erste Soll-Bild abgeleitet wird.

Aus der Verteilung der Abweichungswerte in dem Bild kann eine dreidimensionale Veränderung der inneren Architektur des Zahnmodells 200 und/oder eine Materialzuordnung zu den verschiedenen räumlichen Bereichen ermittelt werden.

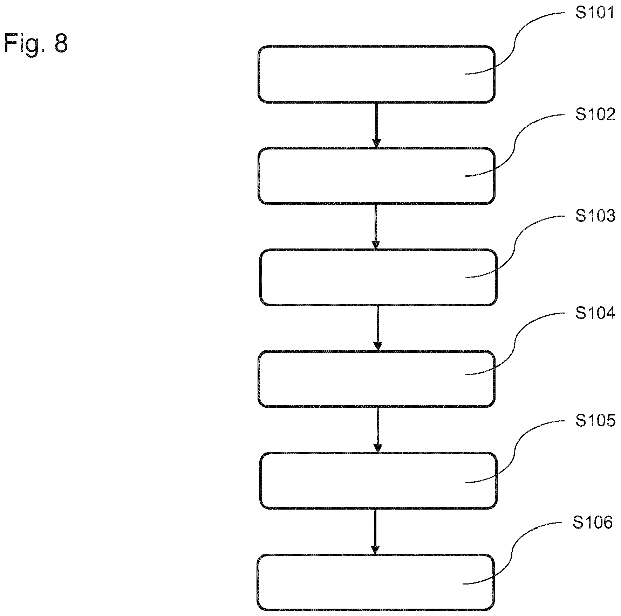

Fig. 8 zeigt ein Blockdiagramm eines Verfahrens zum Vergleichen dreidimensionaler dentaler Strukturen. Im Schritt S101 wird der Soll-Datensatzes DS-S erfasst, der die Form und die optischen Eigenschaften eines natürlichen Zahns wiedergibt. In Schritt S102 wird das digitale Zahnmodell 200 zum Erzeugen eines Ist-Datensatzes DS-I gerendert, der die Form und die optischen Eigenschaften des digitalen Zahnmodells 200 wiedergibt. In Schritt S103 werden der Ist-Datensatz DS-I und/oder der Soll-Datensatz DS-S auf ein gemeinsames Koordinatensystem auf Basis der jeweiligen Form skaliert. In Schritt S104 wird ein erster Vergleichswert aus dem skalierten Ist-Datensatz DS-I an einer Position des Koordinatensystems bestimmt. In Schritt S105 wird ein zweiter Vergleichswert aus dem skalierten Soll-Datensatz DS-S an der gleichen Position des Koordinatensystems bestimmt. Anschließend wird in Schritt

S106 eine Abweichung auf Basis des ersten und des zweiten Vergleichswertes bestimmt.

Der Ist-Datensatzes DS-I und/oder der Soll-Datensatzes DS-S können jedoch auch andere Art und Weise überlagert oder ausgewertet werden, um den ersten und den zweiten Vergleichswert zu erhalten. Der erste Vergleichswert kann an einer gemeinsamen Position in der Überlagerung des Ist- und Soll-Datensatzes DS-I und DS-S erhalten werden, wie beispielsweise ein Farbwert an dieser Position. Der zweite Vergleichswert kann ebenfalls an einer gemeinsamen Position in der Überlagerung des Ist- und Soll-Datensatzes DS-I und DS-S bestimmt werden.

Über das Ist-Bild auf Basis des Ist-Datensatzes DS-I und das Soll-Bild auf Basis des Soll-Datensatzes DS-S wird dasselbe Koordinatensystem gelegt. Dabei kann das Soll-Bild des Zahns dem Ist-Bild des Renderings skalierungsmäßig angepasst werden, um eine maximale Überlagerung und Anpassung zu erreichen. Aus Bildpunkten aus gemeinsamen Bildkoordinaten werden die LAB-Werte verglichen und die Abweichung $\Delta E_{S,I}$ bestimmt.

Als erster und zweiter Vergleichswert können auch Messwerte aus der Mitte oder dem Flächenschwerpunkt der Soll- und Ist-Bilder verwendet werden. In diesem Fall kann auf eine Skalierung auf ein gemeinsames Koordinatensystem verzichtet werden. Es ist auch möglich, Maximalwerte oder Minimalwerte aus dem Ist-Datensatz DS-I und dem Soll-Datensatz DS-S als ersten und zweiten Vergleichswert zu verwenden.

Zum Vergleich können Punkte, Messlinien, Raster, Messfelder (Kacheln), Bereiche wie Zahnhals, Dentin, Schneide oder Farbzonen, dies sich über ähnliche Farbbereiche erstrecken, herangezogen werden. Die kleinste Einheit und somit die genaueste Vorgehensweise ist der Vergleich auf Pixelebene.

Anschließend kann eine dentale Restauration auf Basis desjenigen Ist-Datensatzes hergestellt werden, der weniger als eine vorgegebene Abweichung zu dem Soll-Datensatz aufweist. Hierzu kann eine Herstellungsvorrichtung verwendet werden, wie beispielsweise ein 3D-Drucker, der die dentale Restauration 100 mit den unterschiedlichen Restaurationsmaterialien druckt. Im Allgemeinen können jedoch auch andere Herstellungsvorrichtungen verwendet werden, die die dentale Restauration mit den unterschiedlichen Restaurationsmaterialien herstellen kann.

Für das Verfahren kann eine Computervorrichtung verwendet werden, die die Berechnungsschritte ausführt und anschließend die dentale Restauration 100 mittels der Herstellungsvorrichtung herstellt. Computervorrichtung kann zusätzlich einen Sensor zum Erfassen eines Soll-Datensatzes umfassen. Die Computervorrichtung führt zu diesem Zweck ein Computerprogramm aus, das Befehle umfasst, die bewirken, dass die Computervorrichtung die erforderlichen Verfahrensschritte ausführt. Die Computervorrichtung umfasst einen Prozessor und einen digitalen Speicher, in dem die Datensätze und ein Computerprogramm gespeichert ist, das die Verfahrensschritte ausführt und die Herstellungsvorrichtung geeignet ansteuert.

Durch das Verfahren kann eine Farberfassung einer realen dreidimensionalen dentalen Struktur mit der Farberscheinung einer virtuellen dreidimensionalen dentalen Struktur durchgeführt werden, indem durch eine Überlagerung von (zweidimensionalen) Farbbildern inklusive der Transluzenzwerte der realen und virtuellen dreidimensionalen Struktur beide Strukturen miteinander verglichen werden.

Der Schutzbereich der vorliegenden Erfindung ist durch die Ansprüche gegeben und wird durch die in der Beschreibung erläuterten oder den Figuren gezeigten Merkmale nicht beschränkt.

BEZUGSZEICHENLISTE

100     Zahn
103     Kamera

200     Zahnmodell
203     Linie
205     Raster
207     Bereich
209     Bereich

DS-I     Ist-Datensatz
DS-S     Soll-Datensatz

**Patentansprüche**

**1.**   Verfahren zum Vergleichen dreidimensionaler dentaler Strukturen, mit den Schritten:

- Erfassen (S101) eines Soll-Datensatzes (DS-S), der die Form und die optischen Eigenschaften eines natürlichen Zahns (100) wiedergibt;
- Rendern (S102) eines digitalen Zahnmodells (200) zum Erzeugen eines Ist-Datensatzes (DS-I), der die Form und die optischen Eigenschaften des digitalen Zahnmodells (200) wiedergibt;
- Bestimmen (S104) eines ersten Vergleichswertes aus dem Ist-Datensatz (DS-I);
- Bestimmen (S105) eines zweiten Vergleichswertes aus dem Soll-Datensatz (DS-S); und
- Bestimmen (S106) einer Abweichung ($\Delta E_{S,I}$) auf Basis des ersten und des zweiten Vergleichswertes, **dadurch gekennzeichnet, dass** der Soll-Datensatz (DS-S) so skaliert wird, dass eine Überlagerung mit dem Ist-Datensatz (DS-I) im gleichen Koordinatensystem erzielt wird oder der Ist-Datensatz (DS-I) so skaliert wird, dass eine Überlagerung mit dem Soll-Datensatz (DS-I) im gleichen Koordinatensystem erzielt wird und die ersten und zweiten Vergleichswerte aus einem Bereich des Koordinatensystems erhalten werden.

2. Verfahren nach Anspruch 1, wobei die ersten und zweiten Vergleichswerte entlang eines Rasters oder entlang von Linien innerhalb des Koordinatensystems erhalten werden.

3. Verfahren nach einem der vorangehenden Ansprüche, wobei die Abweichung ($\Delta E_{S,I}$) auf Basis eines euklidischen Abstands des ersten und des zweiten Vergleichswerts bestimmt wird.

4. Verfahren nach einem der vorangehenden Ansprüche, wobei ein Bildrauschen des Soll-Datensatzes (DS-S) und/oder des Ist-Datensatzes (DS-I) durch ein Filter reduziert wird.

5. Verfahren nach einem der vorangehenden Ansprüche, wobei der Soll-Datensatz (DS-S) und/oder der Ist-Datensatz (DS-I) ein zweidimensionales Bild umfasst.

6. Verfahren nach Anspruch 5, wobei der Soll-Datensatz (DS-S) und/oder der Ist-Datensatz (DS-I) mehrere zweidimensionale Bilder aus mehreren Blickwinkeln umfasst.

7. Verfahren nach Anspruch 6, wobei die Abweichung ($\Delta E_{S,I}$) auf Basis mehrerer erster und zweiter Vergleichswerte in unterschiedlichen zweidimensionalen Bildern bestimmt wird.

8. Verfahren nach einem der vorangehenden Ansprüche, wobei der Soll-Datensatz (DS-S) und/oder der Ist-Datensatz (DS-I) eine dreidimensionale Form umfasst.

9. Verfahren nach Anspruch 8, wobei die Abweichung ($\Delta E_{S,I}$) auf Basis mehrerer erster und zweiter Vergleichswerte an den jeweils gleichen Positionen auf der Oberfläche der dreidimensionalen Form bestimmt wird.

10. Verfahren nach einem der vorangehenden Ansprüche, wobei das Koordinatensystem ein zweidimensionales oder ein dreidimensionales Koordinatensystem ist.

11. Verfahren nach einem der vorangehenden Ansprüche, wobei der Soll-Datensatz mittels einer elektronischen Kamera und/oder einem 3D-Scanner erfasst wird.

12. Computervorrichtung zum Vergleichen dreidimensionaler dentaler Strukturen, mit einem Sensor zum Erfassen eines Soll-Datensatzes auf Basis eines natürlichen Zahns, die geeignet ist, das Verfahren nach einem der Ansprüche 1 bis 11 auszuführen.

13. Computerprogramm, umfassend Befehle, die bewirken, dass die Computervorrichtung des Anspruchs 12 das Verfahren nach einem der Ansprüche 1 bis 11 ausführt.

**Claims**

1. A method for comparing three-dimensional dental structures, comprising the steps of:

- acquiring (S101) a target data set (DS-S) representing the shape and optical properties of a natural tooth (100);
- rendering (S102) a digital tooth model (200) to generate an actual data set (DS-I) representing the shape and optical properties of the digital tooth model (200);
- determining (S104) a first comparison value from the actual data set (DS-I);

- determining (S105) a second comparison value from the target data set (DS-S); and

- determining (S106) a deviation ($\Delta E_{S,I}$) based on the first and second comparison values, **characterized in that**

the target data set (DS-S) is scaled such that an overlay with the actual data set (DS-I) in the same coordinate system is achieved or the actual data set (DS-I) is scaled such that an overlay with the target data set (DS-I) in the same coordinate system is achieved and the first and second comparison values are obtained from a region of the coordinate system.

2. The method according to claim 1, wherein the first and second comparison values are obtained along a grid or along lines within the coordinate system.

3. The method according to any one of the preceding claims, wherein the deviation ($\Delta E_{S,I}$) is determined based on a Euclidean distance of the first and the second comparison values.

4. The method according to any one of the preceding claims, wherein an image noise of the target data set (DS-S) and/or the actual data set (DS-I) is reduced by a filter.

5. The method according to any one of the preceding claims, wherein the target data set (DS-S) and/or the actual data set (DS-I) comprises a two-dimensional image.

6. The method according to claim 5, wherein the target data set (DS-S) and/or the actual data set (DS-I) comprises several two-dimensional images from several viewing angles.

7. The method according to claim 6, wherein the deviation ($\Delta E_{S,I}$) is determined based on several first and second comparison values in different two-dimensional images.

8. The method according to any one of the preceding claims, wherein the target data set (DS-S) and/or the actual data set (DS -I) comprises a three-dimensional shape.

9. The method according to claim 8, wherein the deviation ($\Delta E_{S,I}$) is determined based on several first and second comparison values at the same respective positions on the surface of the three-dimensional shape.

10. The method according to any one of the preceding claims, wherein the coordinate system is a two-dimensional or a three-dimensional coordinate system.

11. The method according to any one of the preceding claims, wherein the target data set is acquired by means of an electronic camera and/or a 3D scanner.

12. A computer device for comparing three-dimensional dental structures, comprising a sensor for acquiring a target data set based on a natural tooth, which is suitable for carrying out the method according to any one of claims 1 to 11.

13. A computer program comprising instructions that cause the computer device of claim 12 to perform the method of any one of claims 1 to 11.

**Revendications**

1. Procédé de comparaison de structures dentaires tridimensionnelles, comprenant les étapes suivantes :

   - acquisition (S101) d'un ensemble de données de consigne (DS-S) qui reproduit la forme et les propriétés optiques d'une dent naturelle (100) ;
   - rendu (S102) d'un modèle dentaire numérique (200) pour générer un ensemble de données réelles (DS-I) qui représente la forme et les propriétés optiques du modèle dentaire numérique (200) ;
   - détermination (S104) d'une première valeur de comparaison à partir de l'ensemble de données réelles (DS-I) ;
   - déterminer (S105) une deuxième valeur de comparaison à partir de l'ensemble de données cibles (DS-S) ; et
   - détermination (S106) d'un écart (AE3,1) sur la base de la première et la deuxième valeur de comparaison, **caractérisé en ce que**

l'ensemble de données de consigne (DS-S) est mis à l'échelle de manière à obtenir une superposition avec l'ensemble de données réelles (DS-I) dans le même système de coordonnées, ou l'ensemble de données réelles (DS-I) est mis à l'échelle de manière à obtenir une superposition avec l'ensemble de données de consigne (DS-I) dans le même système de coordonnées et la première et la deuxième valeur de comparaison sont obtenues à partir d'une zone du système de coordonnées.

2. Procédé selon la revendication 1, où la première et la deuxième valeur de comparaison sont obtenues le long d'une grille ou de lignes à l'intérieur du système de coordonnées.

3. Procédé selon l'une des revendications précédentes, où l'écart (AEs,i) est déterminé sur la base d'une distance euclidienne de la première et de la deuxième valeur de comparaison.

4. Procédé selon l'une des revendications précédentes, où un bruit d'image de l'ensemble de données théorique (DS-S) et/ou de l'ensemble de données réel (DS-I) est réduit par un filtre.

5. Procédé selon l'une des revendications précédentes, où l'ensemble de données de consigne (DS-S) et/ou l'ensemble de données réelles (DS- I) comprend une image bidimensionnelle.

6. Procédé selon la revendication 5, où l'ensemble de données de consigne (DS-S) et/ou l'ensemble de données réelles (DS-I) comprend plusieurs images bidimensionnelles sous plusieurs angles de vue.

7. Procédé selon la revendication 6, où l'écart ($\Delta E_{S,I}$) est déterminé sur la base de plusieurs premières et deuxièmes valeurs de comparaison dans différentes images bidimensionnelles.

8. Procédé selon l'une des revendications précédentes, où l'ensemble de données cibles (DS-S) et/ou l'ensemble de données réelles (DS-I) comprend une forme tridimensionnelle.

9. Procédé selon la revendication 8, où l'écart ($\Delta E_{S,I}$) est déterminé sur la base de plusieurs premières et deuxièmes valeurs de comparaison aux mêmes positions respectives sur la surface de la forme tridimensionnelle.

10. Procédé selon l'une des revendications précédentes, où le système de coordonnées est un système de coordonnées bidimensionnel ou un système de coordonnées tridimensionnel.

11. Procédé selon l'une des revendications précédentes, où l'ensemble des données théoriques est acquis au moyen d'une caméra électronique et/ou d'un scanner 3D.

12. Dispositif informatique de comparaison de structures dentaires tridimensionnelles, comprenant un capteur d'acquisition d'un jeu de données de consigne sur la base d'une dent naturelle, apte à mettre en œuvre le procédé selon l'une des revendications 1 à 11.

13. Programme informatique comprenant des instructions pour que le dispositif informatique de la revendication 12 exécute le procédé selon l'une des revendications 1 à 11.

Fig. 1

Fig. 2

Fig. 3

EP 3 984 492 B1

EP 3 984 492 B1

## Fig. 4

LAB$_{XZ\ IST}$
L=84
A=3,5
B=18

Zahnhals

Dentin

Schneide

203

205

207

209

Fig. 5

Fig. 6

DS-S          DS-I

EP 3 984 492 B1

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 199228870 A1
- EP 3530232 A2
- WO 2019023461 A1